# EUROPEAN PATENT APPLICATION

(11) **EP 2 353 597 A1**
(43) Date of publication of application: **10.08.2011**
(21) Application number: 10150691.3
(22) Date of filing: 13.01.2010
(51) Int. Cl.: A61K 31/28, C07F 11/00, A61P 19/02, A61P 29/00

(54) **Arene chromium-carbonyl complexes, method for their preparation of and use thereof as pharmaceuticals**

(71) Applicant: Universität zu Köln, 50923 Köln (DE)
(72) Inventor: Kufer, Thomas, 50354 Hürth (DE); Schmalz, Hans-Günther, 50321 Brühl (DE); Krönke, Martin, 50968 Köln (DE); Velder, Janna, 50321 Brühl (DE); Saiar, Aroonchar, 50931 Köln (DE); Bielig, Harald, 50969 Köln (DE)
(74) Representative: Michalski Hüttermann & Partner Patentanwälte

(57) **Abstract**

The invention relates to novel chromium-carbonyl complexes and a method for the preparation of the latter as well as use of the inventive chromium-carbonyl complexes to inhibit NOD1 and/or NOD2 in the immune system of vertebrates. In a further aspect, the invention relates to the use of the inventive chromium-carbonyl complexes as pharmaceutical active ingredients in a pharmaceutical composition.

## Description

This invention relates to novel arene chromium-tricarbonyl complexes and a method for the preparation of the latter as well as use of the inventive chromium-carbonyl complexes to inhibit NOD1 and/or NOD2 in the immune system of vertebrates. In a further aspect, the invention relates to the use of the inventive chromium-carbonyl complexes as pharmaceutical active ingredient in a pharmaceutical composition.

Polymorphisms in the cytoplasmic pattern recognition receptors NOD1 and NOD2 are linked to chronic inflammatory barrier diseases such as asthma, Blau syndrome, early onset sarcoidosis and Crohn's disease. Chemical substances that specifically modulate NOD1- and NOD2-mediated signaling are thus desirable for scientific and medical interventions.

The innate immune system of vertebrates is the first line of defense reacting to bacterial and viral pathogens. Invariant bacterial motifs, termed pathogen associated molecular patterns (PAMPs), are recognized by specialized pattern recognition receptors (PRRs) to trigger early immune responses. Besides the membrane bound Toll-like receptors (TLRs), C-type lectins (CTLs) and the cytoplasmic RIG-I-like receptors (RLRs), another important class of bacterial sensors, the cytoplasmic nucleotide-binding domain, leucine rich repeat containing family (NLRs) are deemed to be involved in pathogen recognition. Among the best studied members of the NLR family are NOD1 and NOD2, which recognize peptidoglycan subunits. The minimal structure required for activation of NOD1 is the tripeptide L- Ala-D-Glu-mDAP (Tri-DAP), which is present in many Gram-negative as well as in some Gram-positive bacteria. In contrast, NOD2 recognizes muramyl dipeptide (MDP), a motif common for all bacterial peptidoglycans. NOD1/2 share a typical tripartite domain structure consisting of N-terminal caspase activation and recruitment domain (CARD) which mediate signal transduction by protein-protein interactions, a central NACHT domain which mediates activation and oligomerisation of the protein and C-terminal leucine rich repeats (LRRs) which are implicated in pathogen recognition. Recognition of the elicitors initiates homooligomerization and subsequently leads to recruitment of the serine/threonine kinase RIP2 via homophilic interactions of the CARD domains. RIP2 in turn activates the kinase TAK1, which acts on the IKK complex and MAPK pathways. This NOD1/2-mediated activation of NF-κB and MAPKs initiates pro-inflammatory responses that contribute to clearance of bacterial pathogens and modulate adaptive immune responses.

Polymorphisms in NOD1 (CARD4) and NOD2 (CARD15) are linked to chronic inflammatory barrier diseases. An insertion-deletion polymorphism in NOD1 is linked to increased susceptibility to inflammatory bowel disease and asthma. Mutations in NOD2 are involved in Crohn's disease (CD), Blau syndrome (BS), and early onset sarcoidosis (EOS). In the case of CD, three different polymorphisms, located in the C-terminal LRRs of NOD2 lead to a loss-of-function phenotype by affecting the ability of NOD2 to recognize MDP. In contrast, BS and EOS linked mutations in NOD2 display a gain-of-function phenotype causing constitutive activation of NF-κB. NLR dysfunctions can currently only be controlled by influencing the triggered inflammatory responses in a more or less non-specific manner. CD, for example, is treated by inhibition of TNF and the administration of strong anti-inflammatory drugs, which often display severe side effects. No substance that exerts specific inhibitory effects on NOD1 or NOD2 has been reported so far, although the general anti-inflammatory substance curcumin has been proposed to also dampen NOD2-mediated responses.

It is therefore the **object** of the invention to provide a compound which is capable to influence an inflammatory response, especially by at least partly inhibiting NOD1 and/or NOD2.

This object is **solved** by an arene chromium-carbonyl complex according to claim 1, the method of preparation of the latter according to claim 4.

Surprisingly it was found that certain Cr(CO)₃-complexed aromatic compounds carrying a heteroatom substituent in benzylic position very specifically inhibit NOD2-mediated NF-κB signaling. This makes these compounds prominent as means to influence an inflammatory response in the immune system of vertebrates. Furthermore, the inventive arene carbonyl complexes provide insight into the mode of function and structure-activity relationships, i.e. substitution patterns required for NOD2-specific action.

The inventive arene chromium-carbonyl complexes have a general structure according to formula (I) or the respective enantiomer: 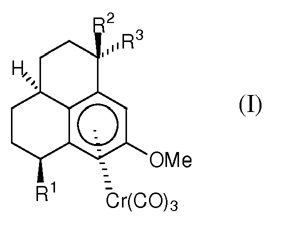 , wherein R¹ is H or an alkyl or alkylene group having 2 to 6 carbon atoms, R² and R³ are independently from each other H, OH, O-R or NH-R. (Only the relative configuration is shown).

It was found, that arene chromium-carbonyl complexes according to the general formula (I) are capable to inhibit MDP-induced NOD2-mediated NF-κB activation in a dose-dependent manner, without causing significant cytotoxic effects.

In a preferred aspect of the invention, R¹ of the arene chromium-carbonyl complex according to formula (I) is a C₄-group, preferably an alkylene-group having 4 carbon atoms.

In a further preferred aspect of the invention, R² or R³ in the arene chromium-carbonyl complex according to formula (I) is a O-CH₂-aryl or a NH-CH₂-aryl group. It should be understood that aryl can be a substituted or unsubstituted phenyl group.

Specific representatives of the inventive arene chromium-carbonyl complexes are the following complexes:

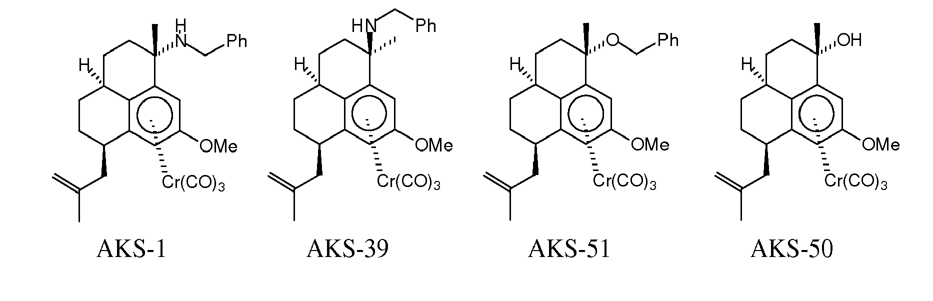

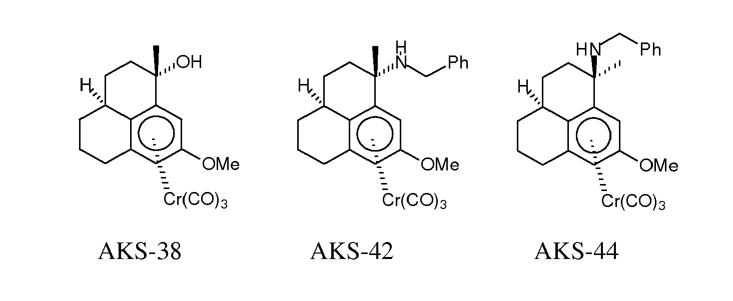

It was found that AKS-01 is a Cr(CO)₃-complexed compound structurally related to the pseudopterosines, compounds isolated from the Caribbean Sea whip *Pseudopterogorgia elisabethae,* that are deemed to exhibit analgesic and anti-inflammatory properties. However, AKS-01 is lacking the glycoside moiety, but carrying a heteroatom substituent in benzylic position. AKS-01 inhibits MDP-induced NOD2-mediated NF-κB activation in a dose-dependent manner. Notably, this substance has virtually no effect on TLR2- and TNF-induced NF-κB activation and only shows a weak influence on NOD1-induced NF-κB activation. Cell viability measurements exclude that the reduction in NF-κB activity is caused by cytotoxic effects.

RIP2 is an essential effector located directly downstream of both NOD1 and NOD2. While not being bound to this theory, it is believed that the inventive arene chromium-carbonyl complexes, like e.g. AKS-1, act upstream of RIP2 in the NOD2 signaling cascade. To test the effect of AKS-01 on RIP2 or downstream components of the signaling pathway, the effect of AKS-01 on RIP2-mediated NF-κB activity induced by overexpression of RIP2 was analyzed. It is found, that NOD2/MDP-induced activation is significantly reduced by AKS-01, used at 15 µM and 30 µM. In contrast, RIP2-mediated NF-κB activation is not significantly affected. These findings strongly suggest that the inventive arene chromium-carbonyl complexes act upstream of RIP2 in the NOD2 signaling cascade.

To substantiate the above results obtained in the HEK293T cell system, the effect of AKS-01 on endogenous NOD2 signaling was examined. In a first step, the human myeloid cell line THP1 was used which is known to be competent in TLR and NOD2 signaling. Cells of the THP1 derivate THP1-Blue, which have a stably integrated secreted alkaline phosphatase (SEAP) NF-κB reporter, were used to monitor NF-κB activation. Cells were treated with AKS-01 or solvent (DMSO) as control and subsequently stimulated with MDP, LPS or a combination of both. As a result it was found that AKS-01 inhibits MDP (NOD2)-induced NF-κB activation in a dose-dependent manner in these cells, whereas LPS (TLR4)-induced NF-κB activation is not affected. Additionally it was revealed that the MDP/LPS synergy is also significantly impaired by AKS-01 in a dose-dependent manner.

In order to expand the analysis, primary dendritic cells (DCs) from C57BL/6 mice were isolated. Cells were treated with 20 µM AKS-01 or treated with DMSO and subsequently activated with 1 µM MDP or 1 µg/ml LPS. NF-κB activation was assessed by p65 (RelA) DNA binding using an ELISA-based method. It was found that the treatment of the DCs with AKS-01 is able to reduce MDP (NOD2)-mediated NF-κB DNA-binding to background levels. In contrast, LPS (TLR4)-induced NF-κB activation is unaffected by AKS-01. These results demonstrate the exclusion of secondary treatment effects accounting for the reduced NF-κB activation. In summary, these experiments demonstrate the selective activity of the inventive arene chromium-carbonyl complexes on endogenous NOD2 in both human and murine cells.

Next, information about the type of inhibition exhibited by AKS-01 was obtained. First, it was tested whether AKS-01 inhibited protein-protein interactions upstream of RIP2. Treatment of the cells with 10 µM AKS-01 did not affect the homo-oligomerization of NOD2. Accordingly, concentrations of 10 µM and 20 µM AKS-01 did not significantly affect the NOD2/RIP2 interaction as judged from co-immunoprecipitation experiments using VSV-tagged RIP2 and FLAG-tagged NOD2. However, at higher concentrations AKS-01 seemed to affect slightly the protein levels of overexpressed RIP2 and NOD2. To get information about the kinetics of the inhibition, MDP was titrated against 20 µM AKS-01 using the NF-κB luciferase reporter assay in HEK293T cells described above. The obtained dose-response curves could be best represented by logarithmic regressions, and show an arrangement expected for a competitive mode of inhibition. Together with the biochemical data that revealed no influence on prominent protein/protein interactions, this strongly supports that AKS-01 acts in a competitive manner in the given experimental setting. It is therefore proposed, that the inventive compounds having a general structure according to formula (I), like e.g. AKS-01, blocks the activation of NOD2 likely by direct binding to the LRR domain of NOD2 or a NOD2-associated MDP receptor protein.

With respect to the method of preparation, the object of the invention is solved by a method for the preparation of an arene chromium-carbonyl complex according to the general formula (I) 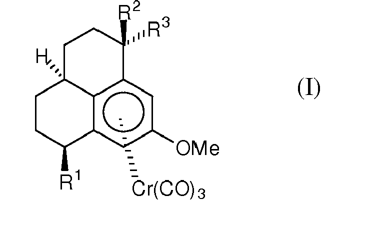 wherein R¹ is H or an alkyl or alkylene group having 2 to 6 carbon atoms, R² and R³ are independently from each other H, OH, O-R or NH-R, said method comprising the steps:
a) preparation of a raceme mixture of a compound having the general formula (II) 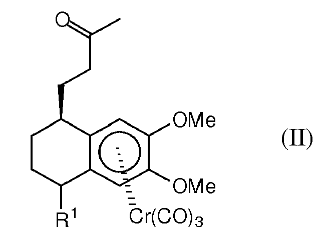 , wherein R¹ is H or an alkyl or alkylene group having 2 to 6 carbon atoms;
b-1) reacting the racemic mixture of step a) with SmI₂ in the presence of a co-solvent such as HMPA, DMPU or TPPA to form a compound according to the general formula (I) wherein R² is H when R³ is OH or R² is OH when R³ is H; or
b-2) reacting the racemic mixture of step a) with a primary amine R⁴-NH₂ to form a compound according to the general formula (III) 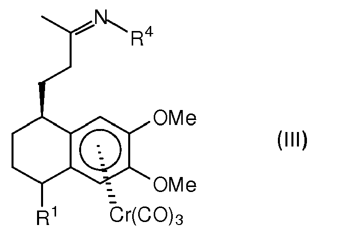 wherein R¹ is H or an alkyl or alkylene group having 2 to 6 carbon atoms, and reacting this compound with SmI₂ in the presence of a co-solvent such as HMPA, DMPU or TPPA to form a compound according to the general formula (I) wherein R² is H when R³ is NH-CH₂-Ph or R² is NH-CH₂-Ph when R³ is H; or
c) reacting the compound formed in step b-1) with an aryl-CH₂-X and a base in the presence of NaH and n-Bu₄NI to form a compound of the general formula (I), wherein R² is H when R³ is O-CH₂-Ph or R² is O-CH₂-Ph when R³ is H.

In the above reaction pathway the primary amine R⁴-NH₂ may be e.g. a benzylamine. The reaction in step b-2) can preferably be performed under water-removing conditions, like e.g. in the presence of alumina.

It is known from the art, that a compound having a structure according to the general formula (II) can be prepared by the following reaction:

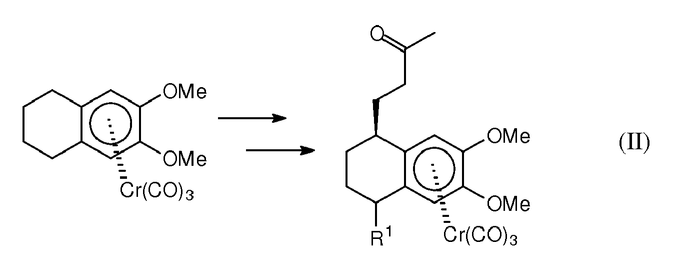

Surprisingly, it was found that a compound having a structure according to the general formula (II), wherein R¹ is H or an alkyl or alkylene group having 2 to 6 carbon atoms, can be transferred into a compound according to the general formula (I), wherein R² is H when R³ is OH or R² is OH when R³ is H, by a cyclization reaction in a moderately polar solvent in the presents of SmI₂ and a co-solvent such as hexamethylphosphoramid (HMPA), DMPU or TPPA.

In an alternative reaction pathway, prior to the cyclization reaction described above, a compound having a structure according to the general formula (II) is transferred into an amine by the reaction of the compound having a structure according to the general formula (II) with benzyl amine in presence of alumina to form a compound according to the general formula (III). Subsequently, the resulting compound according to the general formula (III) can be cyclized by the cyclization reaction described above in the presence of SmI₂. This reaction pathway result in a compound having an inventive general structure according to formula (I), wherein R² is H when R³ is NH-CH₂-Ph or R² is NH-CH₂-Ph when R³ is H.

A further alternative reaction pathway according to the invention results in an inventive compound having an inventive general structure according to formula (I), wherein R² is H when R³ is O-CH₂-Ph or R² is O-CH₂-Ph when R³ is H. Here, the compound resulting from the direct cyclization of the compound having a general structure according to formula (II) is transferred to a compound having a general structure according to formula (I), wherein R² is H when R³ is O-CH₂-Ph or R² is O-CH₂-Ph when R³ is H, by reacting the cyclization product with Ph-CH₂Br in the presence of NaH and n-Bu₄NI. By this reaction, the OH-group in the R² or R³ position is substituted by an aryl-ether group.
Fig. 1 shows the inhibiting effect of AKS-01 on the NOD2-mediated NF-κB activation.
Fig. 2 shows the inhibiting effect of AKS-01 on NOD2 in myeloid cells.
Fig. 3 shows that AKS-01 competes with MDP.
Fig. 4 depicts the chemical structure of some of the inventive compounds.
Fig. 5 depicts a synthesis pathway for the preparation of the inventive compounds.

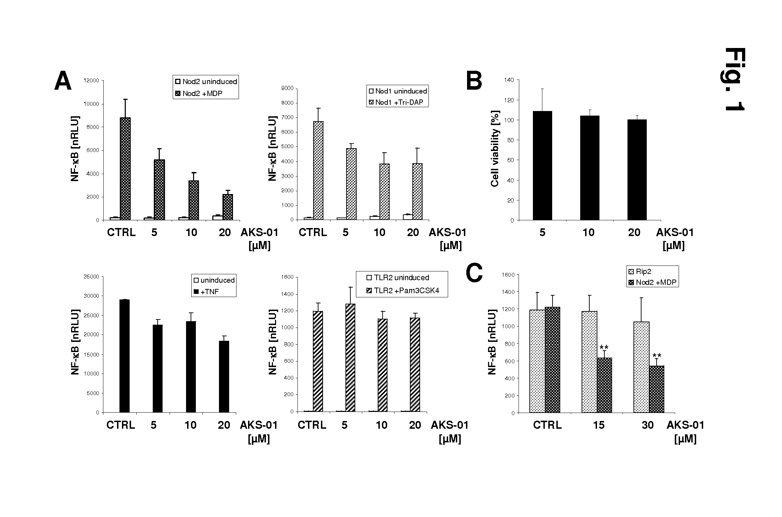

**Figure 1** shows that AKS-01 specifically inhibits NOD2-mediated NF-κB activation. In chart A, HEK293T cells were transfected with NF-κB-luciferase and ß-galactosidase reporter plasmids and with NOD1-, NOD2- or TLR2-expression plasmids and stimulated with 50 nM MDP, 500 nM Tri-DAP, 10 ng/ml TNF, or 0.5 µg/ml Pam3CSK4, respectively. After 15 min of incubation, cells were treated with 5, 10 or 20 µM AKS-01. Cells were lysed after 20 h of incubation, and NF-κB activation and ß-galactosidase activities were measured. Data are given as relative light units normalized with ß-galactosidase values (nRLU). Values are mean +SD (n=3) and are representative of at least two independent experiments. In chart **B,** HEK293T cells were treated with 5 µM, 10 µM, or 20 µM AKS-01. After 20 h, cell viability assays were performed. Data are given in percent living cells of DMSO-treated control (mean +SD, n=3) and are representative of three independent experiments. In chart C, AKS-01 does not affect NF-κB activation upon RIP2 overexpression. HEK293T cells were transfected with NF-κB-luciferase and ß-galactosidase reporter plasmids, cotransfected with RIP2 or NOD2 expression plasmids and left untreated or stimulated with 50 nM MDP, respectively. After 15 min of incubation, cells were treated with 15 µM or 30 µM AKS-01. Cells were lysed after 20 h of incubation and NF-κB activation and ß-galactosidase activities were measured. Mean of nRLU +SD (n=3), representative of two independent experiments are shown. ** p<0.005 (2-tailed t-test).

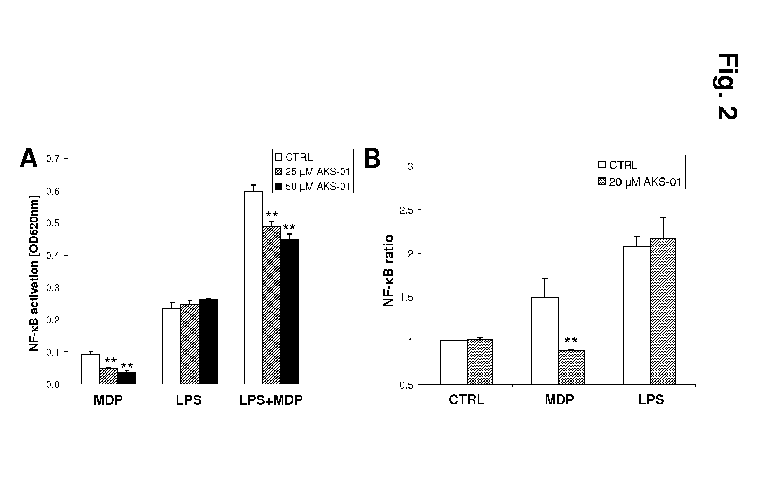

Figure 2 shows that AKS-01 inhibits NOD2 in myeloid cells. In chart **A,** THP1-Blue cells were pre-treated with 25 µM or 50 µM AKS-01 and stimulated with 1 µM MDP, 0.1 µg/ml LPS, or LPS and MDP after 1 h. SEAP-activity was determined after 16 h of incubation. Measurements were performed in triplicates. Data are representative of three independent experiments. In chart **B,** AKS-01 inhibits NOD2-mediated NF-κB activation in primary cells. Murine DCs were left untreated (CTRL) or were stimulated with 1 µM MDP or 1 µg/ml LPS. Samples were subsequently either treated with AKS-01 or left untreated (CTRL). 60 min after stimulation/treatment with AKS-01, nuclear extracts were prepared and a NF-κB ELISA was performed. The stimulation was performed in duplicates. Values are given in fold NF-κB activation, mean +SD (n=4) and are representative of four (THP1) or two (DC) independent experiments. ** p<0.005 (2-tailed t-test).

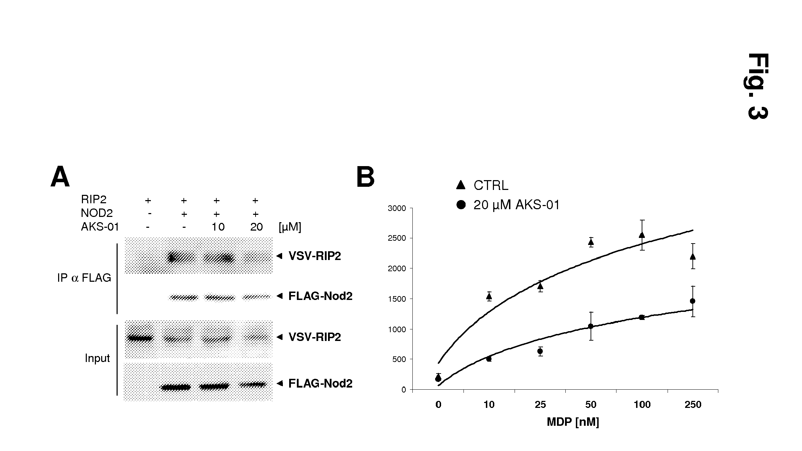

**Figure 3** depicts that AKS-01 competes with MDP. In chart **A,** HEK293T cells were transfected with VSV-RIP2 and FLAG-NOD2 expression plasmids. After 16 h incubation, cells were treated with 10 µM or 20 µM AKS-01 or DMSO as control for 6h. Cells were subsequently lysed and co-immunoprecipitation experiments were performed. Proteins were detected using rabbit anti-RICK and mouse anti-FLAG antibodies. **B,** HEK293T cells were transfected with NF-κB-luciferase and ß-galactosidase reporter plasmids and with a NOD2 expression plasmid and stimulated with the indicated amounts of MDP. After 30 min of incubation, cells were treated with 20 µM AKS-01 or DMSO. Cells were lysed after 20 h of incubation, and NF-κB activation and ß-galactosidase activities were measured. Mean of nRLU, +SD (n=3), representative of two independent experiments are shown. Lines represent logarithmic regressions of each dataset.

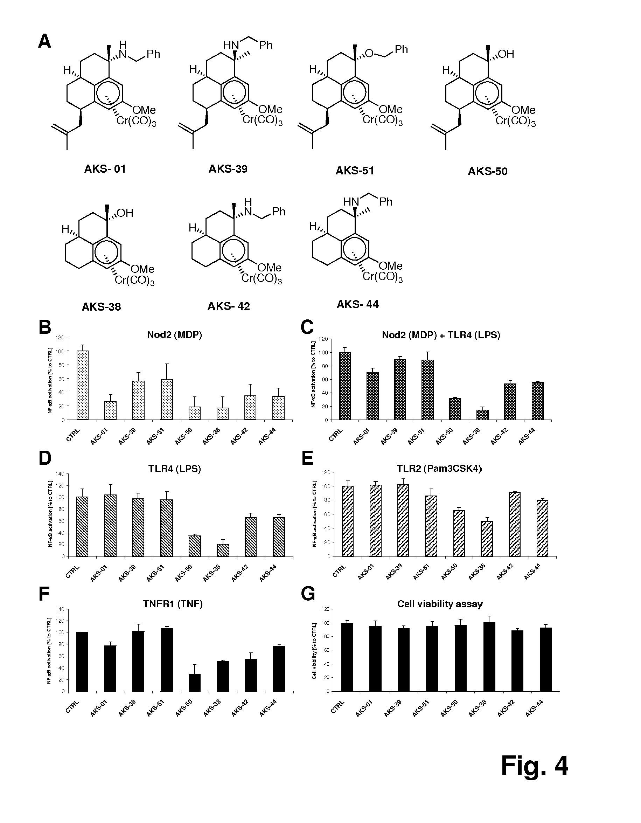

**Figure 4** shows the structural requirements for NOD2-specific effects. In chart A, the chemical structures of the some compounds according to the inventive general formula (I) are shown, i.e. AKS-01 and its derivatives. In charts **B-F,** THPl-Blue cells were pre-treated with 50 µM of the indicated compound or DMSO as control. After 1h, cells were stimulated with 1 µM MDP (B), 0.1 µg/ml LPS (D), 1 µM MDP and 0.1 µg/ml LPS (**C**), 10 ng/ml Pam3CSK4 (**E**), or 100 ng/ml TNF (**F**). SEAP activity was determined after 16 h of incubation. Data are representative of at least two independent experiments (n=3). **G**, THPl-Blue cells were treated with 50 µM of the indicated compound or DMSO. After 16 h a XTT cell viability assay was performed. Data are given in percent living cells of DMSO-treated control. Values are mean +SD (n=3) and are representative of 3 independent experiments.

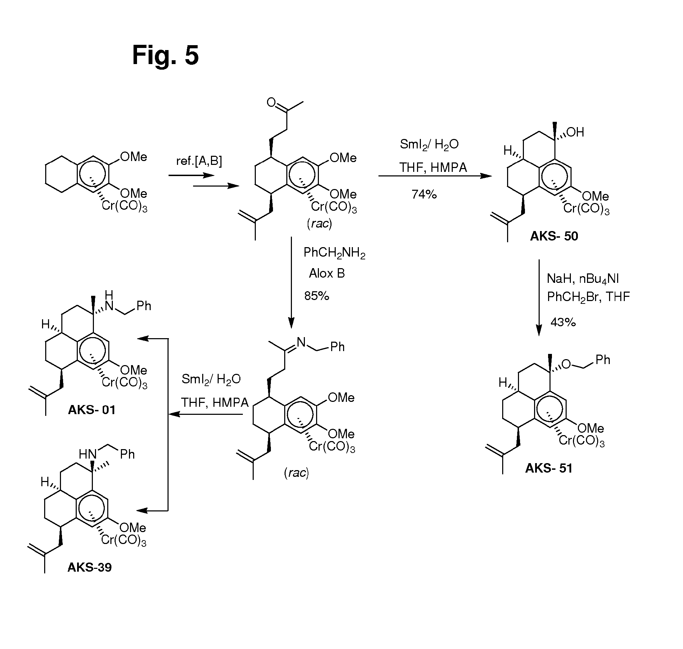

**Figure 5** depicts a pathway for a chemical synthesis of chromium complexes according to the inventive general structure (I), especially for **AKS**-01, AKS-39, **AKS**-50, and **AKS**-51. A related scheme can be used to synthesize compounds **AKS**-38, **AKS**-42, **AKS**-44 lacking the 2-methyl-2-propenyl side chain. All reaction will result in racemic mixtures of the compounds, while the enantiomers can be separated by appropriate methods. In the following, the invention is described in terms of embodiments, while the inventive idea is not limited to the specific embodiments enclosed.

### Preparation of compounds having a general structure according formula (I)

Compounds AKS-01, AKS-38, AKS-39, AKS-42, AKS-44, AKS-50 and AKS-51 were synthesized as detailed below. All tested compounds were diluted in endotoxin free dimethylsulfoxide (DMSO, Sigma D2650). Tri-DAP, MDP, TNF, N-palmitoyl-S-[2,3-bis(palmitoyloxy)-(2RS)-propyl]-[R]-cysteinyl-[S]-seryl-[S]-lysyl-[S]-lysyl-[S]-lysyl-[S]-lysine x 3 HCl (Pam3CSK4) and LPS were purchased from Invivogen. Substances were stored in the dark at -20°C. All chromium complexes were synthesized in racemic form following synthetic schemes published before (compare Figure 5). The purity and identity of all compounds were secured by NMR spectroscopy. NMR data for AKS-50: ¹H NMR (270 MHz, CDCl₃): δ (ppm) = 1.26 (ψdq, 1H, J₁=4.0 Hz, J₂=12.0 Hz), 1.41 (ψdq, 1H, J₁=4.0 Hz, J₂=12.0 Hz), 1.56 (s, 3H), 2.00-2.18 (m, 2H), 2.16 (s, OH), 2.26-2.42 (m, 2H), 2.58 (ψtt, 1H, J₁=4.0 Hz, J₂=12.0 Hz), 2.88-2.98 (m, 1H), 3.70 (s, 3H), 4.76 (s, 1H), 4.89 (s, 1H), 5.07 (s, 1H), 5.42 (s, 1H); ¹³C NMR (67.7 MHz, CDCl₃) : δ (ppm) = 21.9 (CH₃), 24.7 (CH₂), 25.2 (CH₂), 28.9 (CH₂), 32.8 (CH₃), 35.4 (CH), 37.4 (CH), 39.2 (CH₂), 47.3 (CH₂), 55.5 (CH₃), 71.3 (C), 74.8 (CH), 79.0 (CH), 100.9 (C), 113.5 (CH₂), 116.3 (C), 121.6 (C), 142.2 (C), 142.8 (C), 234.3 (s, Cr(CO)₃).

*Preparation of compound AKS-51* (Figure 5): To a solution of AKS-50 (60.0 mg, 0.14 mmol) in tetrahydrofuran (THF) (3 ml) at room temperature NaH (7.4 mg, 0.17 mmol 55% dispersion) was added and the mixture was stirred for 1 h. At that time n-Bu₄NI (11.0 mg, 0.03 mmol) and benzyl bromide (0.17 mmol, 20 µL) were added. The mixture was stirred for 20 h and then quenched by adding saturated NH₄Cl. The mixture was extracted with cyclohexane/EtOAc = 5/1. The organic phases were washed with brine, then dried with MgSO₄ and evaporated *in vacuo* to give a yellow residue. After column chromatography (cyclohexane/EtOAc = 5/1) and recrystallisation from n-pentane 32 mg of AKS-51 were obtained as yellow crystals. NMR data for AKS-51: ¹H NMR (600 MHz, C₆D₆): δ (ppm) = 0.97-1.02 (m, 2H), 1.15 (ψdd, 1H, J₁=4.0 Hz, J₂=13.0 Hz), 1.26 (s, 3H), 1.33 (ψdd, 1H, J₁=4.0 Hz, J₂=13.0 Hz), 1.53 (s, 3H), 1.66 (br.d, 1H, J= 13.0 Hz), 1.72 (br.d, 1H, J= 13.0 Hz), 1.86 (ψtt, 1H, J₁=4.0 Hz, J₂=13.0 Hz), 2.08-2.13 (m, 3H), 2.35-2.42 (m, 1H), 2.87-2.93 (m, 1H), 3.00 (s, 3H), 4.42 (d, 1H, J=11.3 Hz), 4.66 (s, 1H), 4.72 (d, 1H, J=11.3 Hz), 4.78 (s, 1H), 4.79 (s, 1H) 5.45 (s, 1H), 7.12 (t, 1H, J= 7.4 Hz), 7.26 (t, 2H, J= 7.4 Hz), 7.59 (d, 2H, J=7.4); ¹³C NMR (150 MHz, C₆D₆): δ (ppm) = 22.3 (CH₃), 25.6 (CH₂), 26.2 (CH₂), 29.3 (CH₂), 30.8 (CH₃), 34.1 (CH₂), 36.4 (CH), 37.6 (CH), 47.9 (CH₂), 55.5 (CH₃), 65.0 (CH₂), 75.9 (C), 76.2 (CH), 78.9 (CH), 100.2 (C), 114.0 (CH₂), 115.5 (C), 120.4 (C), 128.9 (2 X CH), 129.3(3 X CH), 140.2 (C), 142.5 (C), 143.9 (C), 235.6 (Cr(CO)₃).

### Tissue culture

HEK293T cells were grown at 37°C with 5% CO₂ in Dulbecco's MEM (with 3.7 g/l NaHCO₃, 4.5 g/l D-Glucose and stable glutamine, without Na-Pyruvate, low endotoxin) (Biochrom AG) containing 10% heat-inactivated FCS (BioWest) and penicillin-streptomycin (100 IU/ml and 100 mg/ml, respectively) (Biochrom AG). THPl-Blue cells (purchased from Invivogen) were maintained in very low endotoxin RPMI 1640 (Biochrom AG) containing 10% heat-inactivated FCS (BioWest), penicillin-streptomycin (100 IU/ml and 100 mg/ml, respectively) and 100 µg/ml zeocin (Invivogen). Cells were continuously tested for mycoplasma contamination using PCR.

### Reportergene assays

Activation of NF-κB was measured using a modification of the luciferase reporter assay described in the art. Briefly, 3x104 HEK293T cells were seeded in 96-well plates (TPP) and transfected using Fugene6 (Roche). Per well, 8.6 ng ß-gal-plasmid, 13 ng luciferase-reporter plasmid and 0.1 ng NOD2, 0.5 ng NOD1, 10 ng TLR2 or 5 ng RIP2 expression plasmid were used; samples were adjusted with pcDNA3 to 51 ng DNA total. Cells were stimulated immediately after transfection with 50 nM MDP, 500 nM TriDAP, 10 ng/ml TNF, or 0.5 µg/ml Pam3CSK4 and treated with the indicated amounts of compounds 15 minutes after stimulation. After 16 h of incubation, cells were lysed and the luciferase activity was measured. The luciferase activity was normalized as a ratio to the ß-galactosidase activity. Mean and standard deviations were calculated from triplets.

### Cell viability assays

3x104 HEK293T cells or 1.5x105 THPl-Blue cells were seeded in 96-well plates and treated with the compounds at the indicated concentrations or DMSO as control. After 16 h of incubation, XTT-assays (Cell Proliferation Kit II (XTT), Roche) were performed according to the manufacturer's manual. Measurements were performed in triplicates; data are presented as percent cell viability to the DMSO control (set to 100%) (mean +SD).

### Detection of NF-κB activation in THP1-Blue cells

1.5x105 THP1-Blue cells (purchased from Invivogen) were seeded in 96-well plates, treated with the indicated concentrations of the compounds and incubated for 1 h at 37°C. Cells were subsequently stimulated with 1 µM MDP, 0.1 µg/ml LPS, or MDP and LPS. After 16 h, 20 _{[}tl supernatant of each sample were added to 200 µl QUANTI-Blue medium (Invivogen) and incubated at 37°C. SEAPactivity was determined after 3 h of incubation in a photometer at 620 nm according to the manufacturer's manual. Measurements were performed in triplicates; data are given as percent NF-κB activation to DMSO control (set to 100%) (mean +SD).

### Determination of NF-κB-DNA binding

Dendritic cells (DCs) were generated from mouse bone marrow by adapting a method described in the art. In brief, bone marrow cells isolated from C57BL/6 mice were cultured in RPMI 1640 supplemented with 5 % FBS, 500 U recombinant mouse granulocyte/ macrophage-colony stimulating factor (GM-CSF), 20 µg/ml gentamicin, and 50 µM 2-mercaptoethanol. Medium was exchanged in two-day intervals. DCs were isolated by magnetic cell sorting with a CD11c-specific monoclonal antibody (Miltenyi Biotec). After 7 days of incubation, DCs were treated with the compound or DMSO as a control and subsequently stimulated with either 1 µM MDP for 1 hour or with 1 µg/ml LPS for 15 min or MDP plus LPS for their respective incubation durations. Active p65 was measured using the TransAM™ NF-κB p65 kit (ActiveMotif, Belgium) from nuclear lysates according to the manufacturer's instructions. Biological experiments were conducted in duplicates twice in an independent manner.

### Immunoprecipitation and immunoblotting

For immunoprecipitation, HEK293T cells were transiently transfected, using FuGene6 (Roche) according to the manufacturer's conditions, with the indicated plasmids (1µg plasmid per 6-cm dish) and incubated for 24 h. Cells were lysed in NP-40 buffer (150 mM NaCl, 1% NP-40, 50 mM Tris-HCl, pH 7.5) containing phosphatase inhibitors (20 mM β-glycerophosphate, 5 mM NaF, 100 µM Na₃VO₄, and complete protease inhibitor cocktail [Roche]). Lysates were cleared for 20 min at 14,000 g at 4°C. Immunoprecipitation was subsequently carried out for 4 h at 4°C by adding anti-FLAG beads (M2 gel; Sigma-Aldrich) to the cell extracts. Beads were precipitated by centrifugation steps and washed five times in NP-40 buffer before sodium dodecyl sulfate loading buffer was added. Typically, about 10 times more precipitate than input was loaded into the gel. Proteins were separated by Laemmli sodium dodecyl sulfatepolyacrylamide gel electrophoresis and transferred by semidry Western transfer to a nitrocellulose membrane (Bio-Rad). Proteins were detected by incubation of the membrane subsequently with primary and secondary antibodies and by a final incubation with SuperSignal West Femto maximum sensitivity substrate (Pierce). Signals were recorded using a Fujifilm LAS4000 ECL camera system. Primary antibodies were mouse anti-FLAG M2 (1:1000) (F3165; Sigma-Aldrich) and rabbit anti-RIP2 (1:500) (Cayman Chemical Company, Cat. 160785). Secondary antibodies were horseradish peroxidise (HRP)-conjugated goat anti-mouse IgG (1:4,000) (170-6616; Bio-Rad) and HRP conjugated goat anti-rabbit IgG (1:4,000) (170-6515; Bio-Rad).

## Claims

1. An arene chromium-carbonyl complex, said complex having the general formula (I) 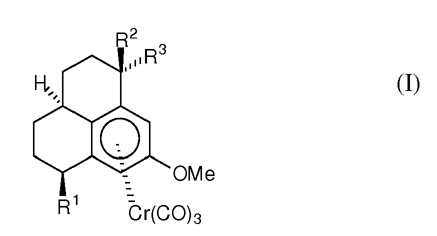 , wherein R¹ is H or an alkyl or alkylene group having 2 to 6 carbon atoms, R² and R³ are independently from each other H, OH, O-R or NH-R.

2. The arene chromium-carbonyl complex according to claim 1, wherein R¹ is a C₄-group, preferably an alkylene-group having 4 carbon atoms.

3. The arene chromium-carbonyl complex according to one of the claims 1 or 2, wherein R² or R³ is a O-CH₂-Ph or a NH-CH₂-Ph group.

4. A method for the preparation of an arene chromium-carbonyl complex according to the general formula (I) 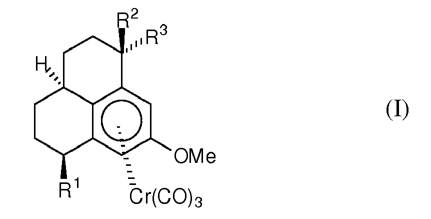 , wherein R¹ is H or an alkyl or alkylene group having 2 to 6 carbon atoms, R² and R³ are independently from each other H, OH, O-R or NH-R, said method comprising the steps:
a) preparation of a raceme mixture of a compound having the general formula (II) 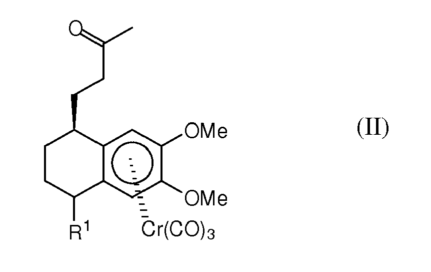 , wherein R¹ is H or an alkyl or alkylene group having 2 to 6 carbon atoms;
b-1) reacting the racemic mixture of step a) with SmI₂ in the present of an co-solvent, preferably HMPA, DMPU or TPPA, to form a compound according to the general formula (I) wherein R² is H when R³ is OH or R² is OH when R³ is H; or
b-2) reacting the racemic mixture of step a) with a primary amine R⁴-NH₂ to form a compound according to the general formula (III) 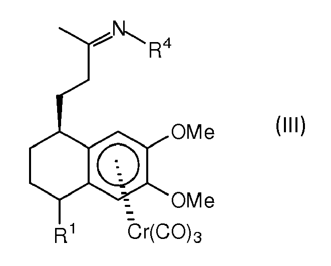 , wherein R¹ is H or an alkyl or alkylene group having 2 to 6 carbon atoms, and reacting this compound with SmI₂ in the presence of a co-solvent, preferably HMPA, DMPU or TPPA to form a compound according to the general formula (I) wherein R² is H when R³ is NH-CH₂-Ph or R² is NH-CH₂-Ph when R³ is H; or
c) reacting the compound formed in step b-1) with Ph-CH₂Br in the presence of NaH and nBu₄NI to form a compound of the general formula (I) wherein R² is H when R³ is O-CH₂-Ph or R² is O-CH₂-Ph when R³ is H.

5. Use of an arene chromium-carbonyl complex according to one of the claims 1 to 4, a salt of it, or a pharmaceutical compatible derivate of it in a pharmaceutical composition.

6. Use of a composition comprising an arene chromium-carbonyl complex according to one of the claims 1 to 4, a salt of it , or a pharmaceutical compatible derivate of it in a pharmaceutical composition as antiphlogistica.

7. The use according to claim 6 to treat a disease caused by a polymorphism of NOD1 or NOD2.

8. The use according to one of the claims 6 and 7 to treat inflammatory bowel disease, asthma, Crohn's disease, Blau syndrome, or early onset sarcoidosis.

9. The use according to claim 6 to treat a chronic inflammatory disease.

10. The use according to claim 9 to treat rheumatoid arthritis.
